Europäisches Patentamt

(19) European Patent Office

Office européen des brevets

(11) EP 0 585 070 B1

(12) **EUROPEAN PATENT SPECIFICATION**

(45) Date of publication and mention
of the grant of the patent:
**24.03.1999 Bulletin 1999/12**

(51) Int. Cl.$^6$: **A61B 8/08**, G01S 7/52

(21) Application number: **93306604.5**

(22) Date of filing: **20.08.1993**

(54) **Enhancement of organ wall motion discrimination**

Verbesserung zur Unterscheidung bei der Organwandbewegung

Amélioration pour la discrimination de mouvement de la paroi d'organe

(84) Designated Contracting States:
**AT BE CH DE DK ES FR GB GR IE IT LI LU MC NL PT SE**

(30) Priority: **21.08.1992 GB 9217830**
**03.11.1992 US 970904**

(43) Date of publication of application:
**02.03.1994 Bulletin 1994/09**

(73) Proprietor:
**EASTMAN KODAK COMPANY
(a New Jersey corporation)
Rochester, New York 14650 (US)**

(72) Inventors:
• **Fitch, Geoffrey
McCordsville, IN 46055 (US)**
• **Feigenbaum, Harvey
Carmel, IN 46032-9688 (US)**

(74) Representative:
**Pohle, Reinhard, Dipl.-Phys. et al
c/o KODAK AKTIENGESELLSCHAFT
Patentabteilung
Hedelfinger Str. 54-60
70327 Stuttgart (DE)**

(56) References cited:
**EP-A- 0 359 130          EP-A- 0 484 181
EP-A- 0 487 339          WO-A-91/19457
AT-B-   343 734**

• **PATTERN RECOGNITION, vol. 18, no. 2, 1985, GB, pages 115 - 124 S.TAMURA ET AL, 'THREE-DIMENSIONAL RECONSTRUCTION OF ECHOCARDIOGRAMS BASED ON ORTHOGONAL SECTIONS'**
• **I.E.E.E. TRANSACTIONS ON BIOMEDICAL ENGINEERING, vol. BME34, no. 3, March 1987, NEW YORK, US, pages 244 - 247 R.S.PRASAD ET AL, 'An Image Processing Method for Cardiac Motion Analysis'**

## Description

This invention relates to the use of diagnostic imaging systems for the diagnosis of diseases of the heart and other vessels and organs of the body and, in particular, to the use of a sequence of images of internal structure of the body for the identification of infirmities in the health of the heart and other vessels and organs.

Various noninvasive diagnostic imaging modalities are capable of producing sequences of cross-sectional images of organs or vessels which depict the condition of such vessels or organs during sequential phases of their operation. An imaging modality which is well suited for such real-time noninvasive imaging is ultrasound. Ultrasonic diagnostic imaging systems are in widespread use for performing ultrasonic imaging and measurements through the use of scanheads which are placed against the skin. Such systems are in common use by radiologists, cardiologists, and obstetricians for examinations of the heart, a developing fetus, or internal abdominal organs. These systems operate by transmitting waves of ultrasonic energy into the body, receiving echoes returned from tissue interfaces upon which the waves impinge, and translating the received echo information into structural representations of the planar "slice" of the body through which the ultrasonic waves are directed.

The ability of ultrasonic diagnostic systems to rapidly produce sequences of images of the cardiovascular system has been especially useful in the practice of cardiology. Cardiologists can rapidly and easily acquire a sequence of images during the cardiac cycle, which can be viewed in real time or stored in Cineloop$^{tm}$ memory for subsequent review, at which time the entire sequence can be replayed at various speeds or individual images examined.

A disease state that can be accurately diagnosed by these means is myocardial infarction or heart attack. The cardiologist will image the heart wall or myocardium to dynamically detect changes in contraction brought about by an ischemic event as a result of coronary artery occlusion. These changes in myocardial motion may range from subtle disturbances in contractility to total akinesia. Differentiating subtle heart wall motion abnormalities arising from partial obstructions are often more difficult than those reflecting changes from complete occlusion.

Echocardiographic imaging has traditionally utilized greyscale imaging techniques, particularly those which provide scan formats capable of imaging the heart transthoracically, through the ribs. In a greyscale presentation, tissue structure is represented in varying shades of grey. Color imaging is a relatively recent tool for cardiologists, and has in recent years been utilized in color Doppler imaging where blood flow velocities are represented in varying hues of color. Structure continues to be shown in greyscale. But the interest in color imaging has been present for two decades. In the early 1970's color was first used to show structure, with hues of color used in place of the shades of grey of the greyscale image. See, e.g., Kossoff, G., "Display techniques in ultrasound pulse echo investigations: a review", J. Clin. Ultrasound 2: 61-72 (1974). This utilization of color was said to be an improvement, in that the human eye was said to perceive finer gradations of color than shades of grey. This claim has been disputed over the years, and it was not until the decade of the 1990's that such implementations became routinely commercially available. Color presentations of tissue structure which provide a distinct clinical advantage have been an elusive objective, at best.

European patent application EP-A-0484181 describes an ultrasonic diagnostic apparatus for forming color Doppler images. A shape parameter interpolating unit calculates shape parameters relating to the interframe time between two image frames. The shape parameters are used to interpolate image frames between original images, and the original images are displayed with the interpolated images inserted between them.

European application EP-A-0487339 describes an ultrasound apparatus wherein an ultrasound reference image of tissue is displayed while concurrently sequentially displaying in partial overlapping alignment therewith a sequence of outlines of B-mode images. A further embodiment described in EP-A-0487339 refers to the display of a sequence of ultrasound images stored in a cineloop frame store.

In accordance with a first aspect of the present invention, there is provided a method of imaging motional characteristics of moving tissue within a body as defined in claim 1.

In accordance with a second aspect of the present invention, there is provided processing apparatus for presenting diagnostic cardiac images as defined in claim 13.

Thus, in a preferred embodiment of the present invention, subtle changes in heart wall motion which may be symptomatic of partial coronary artery occlusion are enhanced by first acquiring a sequence of images of the heart during the cardiac cycle. A particular point in the cardiac cycle is denominated as a reference, such as the start of the cycle. One or more image frames acquired during the reference point in the cardiac cycle are utilized to produce a mask image which is given a unique visual appearance, such as coloring the heart wall of the mask image a first color. The succeeding image frames of the sequence are given a contrasting visual appearance, such as coloring the heart wall with a second color. The mask image is displayed on a screen and the succeeding image frames are then displayed in sequence in the display background. The heart wall motion in the subsequent frames will then be seen to move in relation to the mask image, enabling changes in wall motion to be more easily distinguished.

Cognizance may be taken of the fact that the heart will also translate in position in the chest cavity as it beats. A preferred embodiment of the present invention accounts for this condition by allowing the subsequent images to be translated to the position of the mask image when they are reviewed.

It is recognized that accurate delineation of the tissue of the heart wall will facilitate a more accurate diagnosis. Accordingly, selection of the area of an image which is to be highlighted in contrast to the mask image or the succeeding images may be performed by defining the limits of a range of pixel intensities in the initial greyscale image which are to be contrasted. By changing the range limits the user can adjust the display to accurately delineate the heart wall over a wide variety of conditions of ultrasonic signal reception, processing and image display.

In the drawings:

FIGURES 1a through 1d schematically illustrate a sequence of four images of the heart taken during a cardiac cycle;
FIGURES 2a through 2c schematically illustrate the masking of the images of FIGURES 1b-1d by the image of FIGURE 1a in accordance with the principles of the present invention;
FIGURES 3a through 3c schematically illustrate the masking of sequential images of a heart with wall motion abnormalities;
FIGURE 4 illustrates the relocation of images of a translating heart;
FIGURES 5a and 5b illustrate the selection of the greyscale area of an image for contrast enhancement in accordance with the principles of the present invention; and
FIGURE 6 is a flowchart of the implementation of various features of the present invention for an ultrasound image workstation.

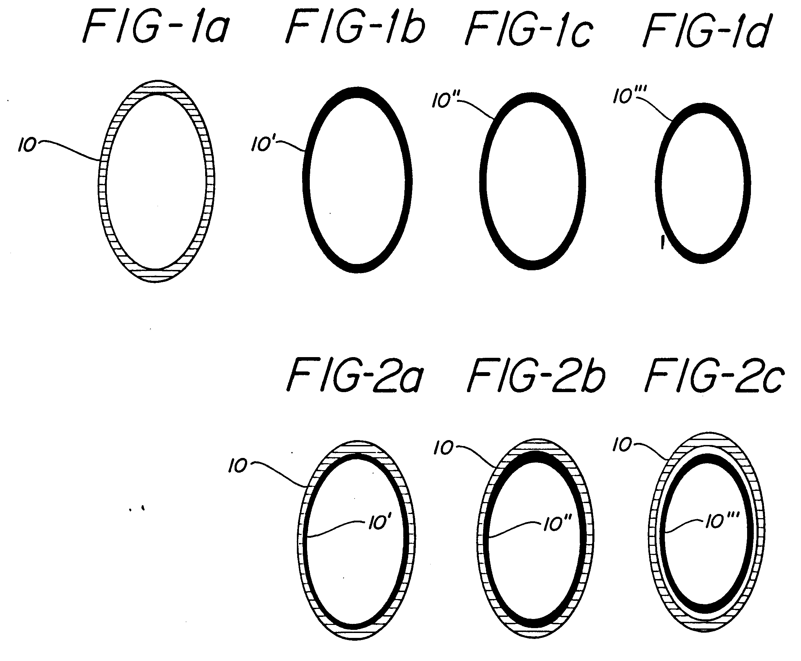

The sequence of images of FIGURES 1a-1d schematically depicts a sequence of images of the heart taken during one heart cycle. For ease of illustration the heart has been schematically represented as an oval. The structure of the heart depicted in the drawings is the myocardium or wall of the heart.

In a preferred application of the present invention a sequence of images of the heart are acquired by an ultrasonic diagnostic imaging system. Each image shows a cross-sectional view of the heart along the plane on which the ultrasonic energy is transmitted through the heart. As is well known, such images may be acquired by use of either a transthoracic scanhead which transmits energy into the body from outside the body, or a transesophageal scanhead positioned within the esophagus. Ultrasonic cardiac images are conventionally acquired and displayed at a rate of approximately 30 frames per second. Thus, sixteen to thirty-two frames could be acquired during a single heart cycle in most cases. The present inventors have found that diagnostically useful information can be determined from an eight frame sequence, and accordingly it is their practice to capture and store in image memory a sequence of eight frames. Each successive frame shows the heart at a successively later phase in the cardiac cycle of contraction and expansion. The clinician may review the continuing display of successive images by activating what is known as the Cineloop™ memory of the system. When activated, the Cineloop memory will display the predetermined number of frames which have been saved. A typical Cineloop memory can store from 64 to 128 successive frames.

FIGURE 1a depicts the heart wall 10 at the very beginning of the cardiac cycle, just before the heart begins its systolic (contraction) phase. This is a preferred reference image for the present invention because the heart is fully expanded at this point of its cycle. This point in the heart cycle is also referred to as end diastole, the end of the heart's relaxive period. The clinician can locate an end diastole image by acquiring an image sequence which is several heart cycles in length, then looking through the sequence until an expansive end diastole image is located. This search may be automated by utilizing an EKG trigger to acquire an image sequence. Since the R-wave trigger of the EKG signal defines end diastole, the R-wave trigger can be used to automatically trigger the Cineloop memory to begin storing successive images, with the first image stored being end diastole. The end diastole image is then used as the basis for the reference, or mask image which is used with a number of successive frames in accordance with the present invention. The total number of successive frames processed in accordance with the invention is preferably eight, for instance.

Preferably the end diastole frame is not used alone, but is digitally averaged or combined with the next successive frame. This is because ultrasound images at end diastole often exhibit signal dropout, and the averaging or combining will fill in image regions experiencing dropout. Alternatively, two successive image frames may be examined on a pixel by pixel basis, and the maximum signal value of the two at each pixel location is used in a consolidated, or superimposed frame. The averaged or superimposed frame is used as the mask image. In a case where signal dropout is not a problem the averaging or superimposition of frames may be omitted.

The mask image is highlighted so as to contrast with the rest of the image frames in the sequence. This may be accomplished by giving the heart wall in the mask frame a distinctive brightness, hue or shading. In a preferred embodiment the heart wall of the mask frame is given a distinctive color such as blue. The blue colorizing of the heart wall 10 is represented by the horizontally lined oval in FIGURE 1a.

When the end diastole image is used to produce the mask frame, the successive images will show the heart contracting as it proceeds through its systolic phase. Hence the ovals of FIGURES 1b-1d are drawn smaller than the oval of FIGURE 1a, although that may not be readily apparent from the individual drawings. In accordance with the present invention these successive images are given a contrasting appearance in brightness, hue or shading. In the preferred embodiment the heart wall 10 of these successive images are colored red. The red coloring is depicted by the dark shading in FIGURES 1b through 1d.

In a review of these images in accordance with the present invention, the mask image of FIGURE 1a is displayed continuously on the screen, and successive images are successively displayed overlaid with the mask image. In the preferred embodiment the mask image is displayed in the foreground as the dominant image and the successive images are displayed in the background. This display enables the clinician to view the motion of the heart wall over a succession of images in constant relation to the reference mask image. Thus, FIGURE 2a shows the concurrent display of the FIGURE 1a and FIGURE 1b images. Since the heart is just beginning to contract in FIGURE 1b, the heart wall 10' is just beginning to appear inside the contrasting heart wall 10 of the mask image. FIGURE 2b shows the mask image displayed with the next successive image of FIGURE 1c, and the heart wall 10" is seen to be making a greater appearance inside the mask image heart wall 10. FIGURE 2c, which shows the concurrent display of the FIGURE 1d image with the mask image, shows the heart wall 10''' now fully inside the heart wall 10 of the mask image.

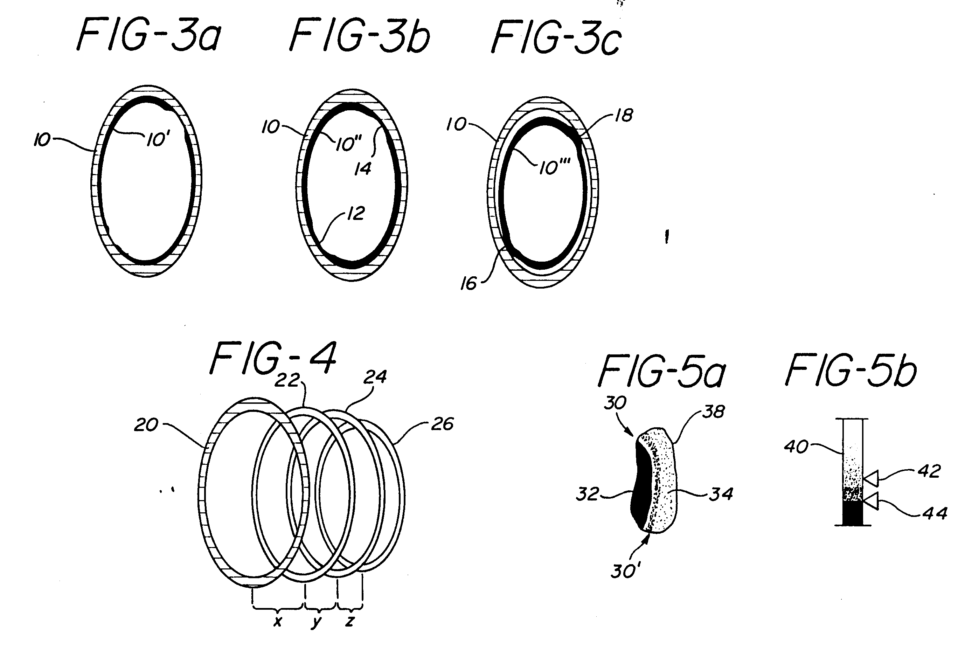

In the operation of a normal healthy heart, the edge of the contracting heart wall will begin to appear evenly inside the heart wall 10 of the mask image. FIGURES 3a-3c depict the operation of a heart where areas of the wall move unevenly or lethargically as a result of coronary artery occlusion. FIGURE 3a shows the first successive image 10' initially appearing unevenly inside the boundary of the mask image wall 10. As the drawing begins to reveal, there are areas of uneven motion at the upper right and lower left of the oval. In FIGURE 3b the next successive image makes these area 12 and 14 of uneven motion even more apparent. In FIGURE 3c the later successive image again highlights the uneven motion, as the wall 10''' of the later image is touching the mask image wall 10 at areas 14 and 16 after the rest of the contracting heart wall 10''' has moved inside the boundary of the mask image 10.

By displaying the images of the sequence in concert with the fixed mask image at display rates up to real time, the dynamics of variations in heart wall motion are viewed in relation to the heart wall position in the mask image, enabling the detection of subtle abnormalities in the performance of the heart. The patient can then be appropriately treated for any suspected partial occlusion of the coronary artery system.

In many patients the heart does not simply expand and contract about a fixed central point of the organ. Rather, the heart itself will translate in its entirety within the chest cavity as it beats. Through observation clinicians have recognized that in certain apical views the heart should appear to move up and down but not right-to-left. In particular, as the heart muscle contracts, the annulus around which the mitral valve is attached should appear to move up and down but not right-to-left in these views. Accordingly it is desirable to correct for any right-to-left translation of the heart annulus in such images before using the sequence of images for diagnosis.

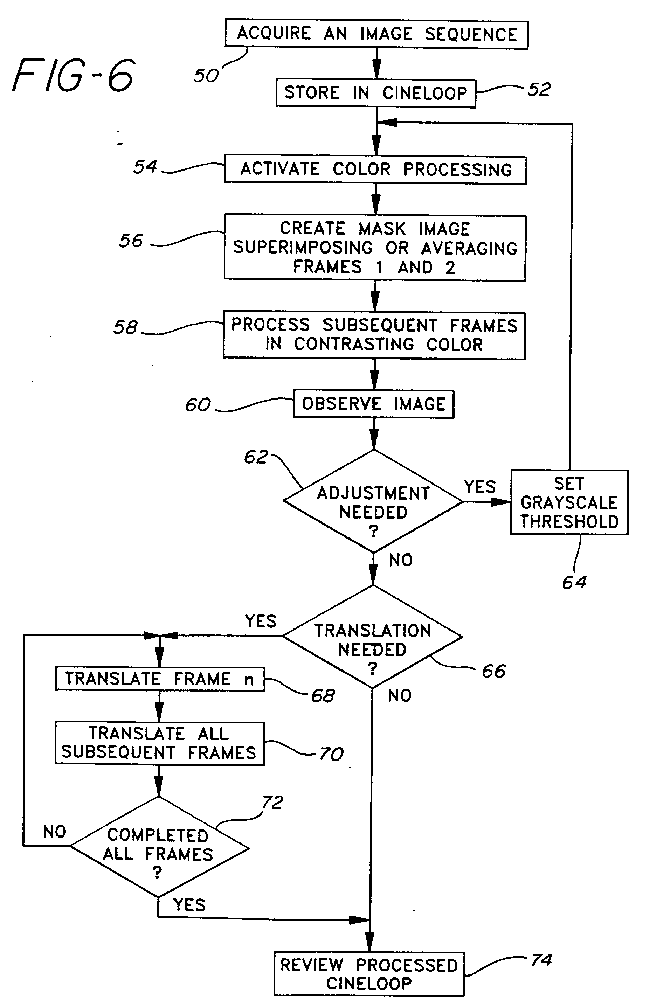

FIGURE 4 illustrates the effect of such heart translation on an image sequence. From the resting position of mask image 20, the heart could move laterally as it contracts to a subsequent position shown by the next heart wall image 22. The new position is displaced from the initial position by an increment x. As the heart continues to contract it could move even further to the right, as indicated by subsequent images 24 and 26. The position of the heart wall in image 24 is displaced from the position of the heart wall in image 22 by a further incremental position y, and the position of the heart wall moves a further increment z by the time of acquisition of image 26. Since this translational movement and resultant disruption of concentric alignment of the heart wall images will have a deleterious effect upon the diagnostic efficacy of the display of overlaid images, the preferred embodiment of the present invention provides for correction of the translational effect. With the first or mask image 20 statically displayed on a screen, the next image 22 in the sequence is concurrently displayed. A control such as a key, a joystick, or a trackball is manipulated to move the heart wall of image 22 to the left by the increment x, bringing the two heart wall images into approximate concentric alignment. As the image 22 is moved, the subsequent images in the sequence are all adjusted by the same amount. Thus, if the sequence comprised a total of eight images, as the second image is aligned with the first image, images three through eight would all be moved by the increment x. When the first and second images have been aligned, the second image 22 is statically displayed concurrently with the third image 24. Since the heart walls of these two images are misaligned by the increment y, the control is used to correct the position of the heart wall of image 24 by this further increment. As before, the remaining images in the sequence are automatically relocated by the increment y. The image 26 is then adjusted by the increment z to bring it into alignment with image 24, in the same manner. At this point, all four images of FIGURE 4 have been aligned, image 22 by the increment x, image 24 by the increment x+y, and image 26 by the increment x+y+z , and all subsequent images in the sequence have been corrected by the total increment of x+y+z . After all eight images in the above example have been aligned in this manner, each stored image is tagged with a display parameter which is the cumulative adjustment to be used in the display of the associated image. The full sequence can be reviewed as above and the diagnosis performed.

It may be appreciated that while the above example of translational correction involves only a simple adjustment in horizontal positioning, correction in a particular embodiment may involve relocation in any direction in the Cartesian coordinate plane of the image, or angular or rotational correction of image position.

As it is the purpose in the illustrated embodiments to display specific tissue in contrasting appearances so as to detect subtle differences in the motion of the tissue, it may be appreciated that consistent identification of the tissue and its boundaries from image to image is important. It would be undesirable to extend the colorizing in the image beyond or short of the actual physical boundary of the heart wall muscle. In accordance with a further aspect of the present invention, a consistent means is provided for colorizing or contrasting that part of the diagnostic image which is identified as the heart wall muscle. Referring to FIGURE 5a, a portion 38 of a diagnostic image of the heart wall is shown. The band of moderate grey between arrows 30-30' represents the cross-sectional display of the heart wall. Inside the heart wall is a dark area 32 which is the display of blood inside the heart. Outside the heart is a lighter grey area 34 of soft tissue. A clinician viewing this image statically can distinguish the moderate grey area of the heart wall and will recognize that the display pixels in this area are to be colorized or contrasted in use of the present invention. To select the band of grey between arrows 30-30' for colorizing, the user views a concurrently displayed greyscale bar 40 as shown in FIGURE 5b. The greyscale bar 40 continuously varies in greyscale shading from the darkest shade to the lightest shade of the image. The clinician then adjusts two slide controls on the system which move upper and lower pointers 42 and 44 along the greyscale bar. The pointers are adjusted until the band of the greyscale between them is the same as the grey shading of the heart wall in the image. When the clinician believes that the pointers are properly set, the command is given to colorize the heart wall in accordance with the range of grey defined between the upper and lower pointers.

The colorized image is then carefully inspected to determine that only the heart wall area of the display has been colorized and that the color does not extend beyond the heart wall pixels in the image. Some further adjustment of the slide controls may then be performed until the colorized area accurately defines the heart wall. The selected range of grey between the pointers is then used to colorize the heart wall on all of the images in the sequence. By reviewing the images and making further adjustment to the pointers 42 and 44 the colorization is finely adjusted to accurately colorize the heart wall even in the presence of varying or weak ultrasonic signal conditions.

In a preferred embodiment of the present invention the parameters controlled by the slide controls and their pointers 42 and 44 are as follows. Pointer 44 defines a greyscale pixel intensity level below which pixels are eliminated from the mask image. The effect of this control is to remove clutter and distractive artifacts from the mask image. This low level pixel elimination effect can also be applied to the underlying images, if desired. Pixels having intensities ranging above the level of the upper pointer 42 are given a constant mask intensity value. Pixels having intensities ranging between the two pointer levels are given color intensities in accordance with their greyscale intensity values, thereby preserving the structural tissue characteristics of the heart wall image.

In some imaging applications the heart wall muscle will expectedly be displayed with the highest intensity in the image. In such an application only a single threshold need be set, above which the image is colored to distinguish the heart wall and below which it is not colored. When the clinician views such an image, a single control such as a slide bar, arrow or function key or numerical threshold value is changed to color more or fewer pixels as the heart wall. The control is manipulated until the clinician is satisfied as to the allocation of distinguishing color to the heart wall muscle in the mask image.

If desired, a second bar similar to greyscale bar 40 can be displayed, the second bar displaying a spectrum of colors. The second bar would have two pointers similar to those of bar 40. In the case of the color bar, a first pointer designates the color to be used for the mask image and a second pointer designates the contrasting color to be used for all of the subsequent images in the sequence. The color intensities may then be modulated as described above.

It is further understood that many ultrasound systems also allow the user to vary the entire greyscale range in which images are displayed, and the foregoing selection of colorized shades may be performed in concert with greyscale adjustment of the entirety of each image in the full sequence.

FIGURE 6 illustrates the flowchart of an implementation of the diagnostic enhancement technique of the present invention for an Imagevue(tm) or ImageVue DCR(tm) workstation available from Nova MicroSonics of Mahwah, New Jersey. At the starting block 50 in the flowchart a sequence of cardiac images is acquired from a video source. The video source could be a videotape on which a sequence of cardiac images has been recorded. The video source could also be the image data port of an ultrasound system which is acquiring the image sequence in real time, such as an Ultramark 9 HDI(tm) ultrasound system available from Advanced Technology Laboratories, Inc. of Bothell, Washington. In either case the images of the sequence are acquired by a video frame grabber and stored in Cineloop(R) memory in the workstation. The ImageVue DCR workstation has a Cineloop memory with a capacity of 64 megabytes, which is capable of storing approximately one thousand image frames. Since a full cardiac cycle can generally be represented by approximately thirty frames, a smaller Cineloop memory may also be adequate for other applications.

In block 54 the color processing of the image sequence is activated by the workstation's operating program. The first operation is to create the mask image. An enhanced image is created by the averaging or superimposition of the first two frame of the image sequence, starting from the chosen reference in the cardiac cycle such as end diastole. The heart wall of the enhanced image is then colored blue as described previously. In block 58 the subsequent frames in the image sequence are distinguished by coloring the heart wall in those frames red.

In block 60 the user observes the images, generally starting with the mask image, to determine if any adjustment in heart wall definition is needed as indicated by decision block 62. If the definition of the heart wall is not acceptable, the grayscale threshold pointers of FIGURE 5b are adjusted and the color processing sequence is repeated with the heart wall colorized in accordance with the new threshold settings as previously described.

Once the heart wall in the images is clearly defined, the workstation proceeds to decision block 66, which poses the question of the need for translation. If the user decides that translation of the image is needed in order to compensate for motion of the heart within the chest cavity, the frame following the mask image is translated into alignment with the heart wall in the mask image as described above. This operation is represented by block 68, starting with frame n where n is equal to two. Alignment of the second frame automatically results in corresponding translation of the subsequent frames as shown by block 70. This translation process continues until all n frames in the sequence have been brought into alignment as indicated by the loop to block 68 from decision block 72. The processed image sequence is now available for review by the user as indicated by block 74.

## Claims

1. A method of imaging motional characteristics of moving tissue within a body, the method comprising:

   (a) acquiring (50) in correct temporal sequence a series of temporally distinct images (10, 10', 10", 10'''; 20 ,22 ,24 ,26) of said tissue during a period of its motional activity; and
   (b) providing (56) tissue represented in a selected image (10;20) in the series with a first predetermined visual appearance;
   (c) providing (58) tissue shown in other images (10' ,10" ,10'''; 22, 24, 26) in the series with a contrasting visual appearance; and

   (d) displaying (74) said selected image (10;20) while concurrently sequentially displaying said other images (10', 10", 10'''; 22, 24, 26) in at least partial overlapping alignment with said vertical image.

2. A method as claimed in claim 1, wherein said other images (10', 10", 10'''; 22, 24, 26) are displayed as a substantially real time sequence.

3. A method as claimed in claim 1 or claim 2, wherein step (b) comprises the step (56) of colouring the tissue shown in said selected image (10;20) with a first color or hue and wherein step (c) comprises the step (58) of coloring the tissue shown in the other images (10', 10", 10'''; 22, 24, 26) in the series with a contrasting color or hue.

4. A method as claimed in any preceding claim, wherein the selected image (10;20) is sequentially displayed in overlapping alignment with each of said other images (10', 10", 10'''; 22, 24, 26).

5. A method as claimed in any preceding claim, wherein said selected image (10;20) is statically displayed while concurrently sequentially displaying said other images (10', 10", 10'''; 22, 24, 26).

6. A method as claimed in any preceding claim, wherein said moving tissue comprises the wall of a beating heart, and wherein said selected image (10;20) represents a view of said beating heart at a predetermined point in the heart cycle.

7. A method as claimed in claim 6, wherein said predetermined point in the heart cycle comprises end diastole.

8. A method as claimed in any preceding claim, wherein said series of images (10', 10", 10'''; 22, 24, 26) of step (a) are acquired ultrasonically.

9. A method as claimed in any one of claims 6 to 8, further comprising substantially concentrically aligning each of the heart images (10', 10", 10'''; 22, 24, 26) with respect to at least one other heart image in the series.

10. A method as claimed in claim 9, wherein the alignment step comprises the step of translating each of the other heart images (22, 24, 26) in said series into overlapping alignment with a selected one (20) of said heart images (20, 22, 24, 26) .

11. A method as claimed in claim 10, wherein the translation of one (e.g. 22) of said other heart images (22, 24, 26) into overlapping alignment with said selected one (20) of said heart images (20, 22, 24, 26) also effects an overlapping alignment of at least one other heart image (e.g. 24) with said selected one (20) of said heart images (20, 22, 24, 26).

12. A method as claimed in any preceding claim, wherein step (a) comprises the step of ultrasonically acquiring a series of greyscale images (10', 10", 10'''; 22, 24, 26) of said tissue during a period of its motional activity; and comprising the further step of selecting a range of greyscale shades which are to be colored.

13. Processing apparatus for presenting diagnostic cardiac images, the apparatus comprising:

means for acquiring in correct temporal sequence a series of cardiac images (10', 10", 10'''; 22, 24, 26) during a period of its motional activity; characterized in that the apparatus further comprises:
means for creating from a selected image in the series a mask image (10;20) in which the heart wall is represented in a first color;
means for representing the heart wall in a plurality of other images (10', 10", 10'''; 22, 24, 26) in the series in a second color; and
means (Fig. 3a-3c) for displaying said mask image (10;20) while concurrently sequentially displaying said other images (10', 10", 10'''; 22, 24, 26)in at least partial overlapping alignment with said mask image.

14. Processing apparatus as claimed in claim 13, further comprising means (40, 42, 44) for determining the portions of said acquired images (10', 10", 10'''; 22, 24, 26) of said series which are to be represented in color.

15. Processing apparatus as claimed in claim 14, wherein said means for acquiring is arranged to acquire a greyscale cardiac image series, and wherein said means for determining (40, 42, 44) is arranged to designate a range of greyscale intensities which are to be represented in color.

16. Processing apparatus as claimed in any one of claims 13 to 15, further comprising means for translating the heart wall representation of images (22, 24, 26) subsequent in the series to said mask image (20) into alignment with the heart wall representation of said mask image (20).

**Patentansprüche**

1. Verfahren zum Abbilden von Bewegungskennwerten eines sich in einem Körper bewegenden Gewebes, **gekennzeichnet durch** die Schritte:

a) Erzeugen (50) in einer genauen zeitlichen Folge einer Reihe von zu unterschiedlichen Zeitpunkten gemachten Aufnahmen (10, 10', 10", 10''', 20, 22, 24, 26) des Gewebes in einer Phase aktiver Bewegung,
b) Versehen (56) des in einer ausgewählten Aufnahme (10; 20) dargestellten Gewebes der Reihe mit einem ersten vorbestimmten visuellen Aussehen,
c) Versehen (58) des in anderen Aufnahmen (10', 10", 10'''; 22, 24, 26) dargestellten Gewebes der Reihe mit einem sich davon unterscheidenden visuellen Aussehen und
d) Darstellen (74) der ausgewählten Aufnahme (10; 20) bei gleichzeitiger aufeinanderfolgender Wiedergabe der anderen Aufnahmen (10', 10", 10'''; 22, 24, 26) in zumindest teilweise überlappender Ausrichtung mit der ausgewählten Aufnahme.

2. Verfahren nach Anspruch 1, dadurch gekennzeichnet, daß die anderen Aufnahmen (10', 10", 10'''; 22, 24, 26) im wesentlichen in Echtzeitfolge dargestellt werden.

3. Verfahren nach Anspruch 1 oder 2, dadurch gekennzeichnet, daß Schritt b) den Schritt (56) umfaßt, bei dem das in der ausgewählten Aufnahme (10; 20) gezeigte Gewebe mit einer ersten Farbe oder einem ersten Farbton eingefärbt wird und Schritt c) den Schritt (58) umfaßt, bei dem das in den anderen Aufnahmen (10', 10", 10'''; 22, 24, 26) gezeigte Gewebe der Reihe mit einer anderen Farbe oder einem anderen Farbton eingefärbt wird.

4. Verfahren nach einem der vorhergehenden Ansprüche, dadurch gekennzeichnet, daß die ausgewählte Aufnahme (10; 20) nacheinander in überlappender Ausrichtung mit jeder der anderen Aufnahmen (10', 10", 10'''; 22, 24, 26) dargestellt wird.

5. Verfahren nach einem der vorhergehenden Ansprüche, dadurch gekennzeichnet, daß die ausgewählte Aufnahme (10; 20) statisch dargestellt wird, während die anderen Aufnahmen (10', 10", 10'''; 22, 24, 26) gleichzeitig aufeinanderfolgend dargestellt werden.

6. Verfahren nach einem der vorhergehenden Ansprüche, dadurch gekennzeichnet, daß das sich bewegende Gewebe die Wand eines schlagenden Herzens umfaßt und daß die ausgewählte Aufnahme (10; 20) eine Ansicht des schlagenden Herzens an einem vorgegebenen Punkt des Herzzyklus darstellt.

7. Verfahren nach Anspruch 6, dadurch gekennzeichnet, daß der vorgegebene Punkt des Herzzyklus eine Enddiastole aufweist.

8. Verfahren nach einem der vorhergehenden Ansprüche, dadurch gekennzeichnet, daß die Aufnahmenfolge (10', 10'', 10'''; 22, 24, 26) gemäß Schritt a) mit Ultraschall erzeugt wird.

9. Verfahren nach einem der Ansprüche 6 bis 8, dadurch gekennzeichnet, daß jede der Aufnahmen (10', 10'', 10'''; 22, 24, 26) vom Herzen mit mindestens einer anderen Aufnahme der Folge im wesentlichen konzentrisch ausgerichtet ist.

10. Verfahren nach Anspruch 9, dadurch gekennzeichnet, daß beim Ausrichten jede der anderen Aufnahmen (22, 24, 26) der Folge translatorisch in überlappende Ausrichtung mit einer ausgewählten Aufnahme (20) der Aufnahmen (20, 22, 24, 26) vom Herzen bewegt wird.

11. Verfahren nach Anspruch 10, dadurch gekennzeichnet, daß die translatorische Bewegung einer (beispielsweise 22) der anderen Aufnahmen (22, 24, 26) vom Herzen in überlappende Ausrichtung mit der ausgewählten Aufnahme (20) der Aufnahmen (20, 22, 24, 26) vom Herzen bewirkt, daß mindestens eine andere Aufnahme (beispielsweise 24) in überlappende Ausrichtung mit der ausgewählten Aufnahme (20) der Aufnahmen (20, 22, 24, 26) vom Herzen gelangt.

12. Verfahren nach einem der vorhergehenden Ansprüche, dadurch gekennzeichnet, daß bei Schritt a) mit Ultraschall eine Reihe von Graustufenaufnahmen (10', 10'', 10'''; 22, 24, 26) des Gewebes in einer Phase aktiver Bewegung erzeugt wird und daß eine Palette von einzufärbenden Graustufenschatten ausgewählt wird.

13. Verarbeitungsvorrichtung zum Darstellen von Aufnahmen vom Herzen für diagnostische Zwecke, mit

   - Mitteln zum Erzeugen in einer genauen zeitlichen Folge einer Reihe von Aufnahmen (10, 10', 10'', 10''', 20, 22, 24, 26) vom Herzen in einer Phase aktiver Bewegung, **gekennzeichnet durch**
   - Mittel zum Erzeugen einer Maskenaufnahme (10; 20) anhand einer ausgewählten Aufnahme der Reihe, bei der die Herzwand in einer ersten Farbe dargestellt ist,
   - Mittel zum Darstellen der Herzwand in einer Vielzahl anderer Aufnahmen (10, 10', 10'', 10''', 20, 22, 24, 26) der Reihe in einer zweiten Farbe und
   - Mittel (Fig. 3a - 3c) zum Darstellen der Maskenaufnahme (10; 20) bei gleichzeitiger aufeinanderfolgender Darstellung der anderen Aufnahmen (10', 10'', 10'''; 22, 24, 26) in zumindest teilweise überlappender Ausrichtung mit der Maskenaufnahme.

14. Verarbeitungsvorrichtung nach Anspruch 13, gekennzeichnet durch Mittel (40, 42, 44) zum Bestimmen der Abschnitte der erzeugten Aufnahmen (10', 10'', 10'''; 22, 24, 26) der Reihe, die in Farbe wiedergegeben werden sollen.

15. Verarbeitungsvorrichtung nach Anspruch 14, dadurch gekennzeichnet, daß die Mittel zum Erzeugen derart angeordnet sind, daß sie eine Reihe von Graustufenaufnahmen vom Herzen erzeugen und daß die Mittel zum Bestimmen (40, 42, 44) derart angeordnet sind, daß sie einen Bereich von Graustufenintensität bestimmen, der in Farbe wiedergegeben werden soll.

16. Verarbeitungsvorrichtung nach einem der Ansprüche 13 bis 15, dadurch gekennzeichnet, daß Mittel vorgesehen sind, die die in der Reihe aufeinanderfolgenden Aufnahmen (22, 24, 26) mit der Wiedergabe der Herzwand translatorisch zur Maskenaufnahme (20) verschieben derart, daß sie mit der Wiedergabe der Herzwand in der Maskenaufnahme (20) ausgerichtet sind.

**Revendications**

1. Procédé de formation d'images de caractéristiques de mouvement d'un tissu se déplaçant à l'intérieur d'un corps, le procédé comprenant les opérations consistant à :

   (a) acquérir (50), en une séquence temporelle correcte, une série d'images temporellement distinctes (10, 10', 10'', 10'''; 20, 22, 24, 26) dudit tissu pendant une période de son activité de mouvement; et
   (b) munir (56) le tissu représenté dans une image sélectionnée (10 ; 20) dans la série, d'une première apparence visuelle prédéterminée;
   (c) munir (58) le tissu représenté dans les autres images (10', 10'', 10'''; 22, 24, 26) dans la série, d'une apparence visuelle contrastée; et
   (d) afficher (74) ladite image sélectionnée (10 ; 20) tout en affichant séquentiellement, simultanément, lesdites autres images (10', 10'', 10''' ; 22, 24, 26) en au moins un alignement en chevauchement partiel avec ladite image sélectionnée.

2. Procédé selon la revendication 1, dans lequel lesdites autres images (10', 10'', 10'''; 22, 24, 26) sont affichées comme une séquence sensiblement en temps réel.

3. Procédé selon la revendication 1 ou la revendication 2, dans lequel l'étape (b) comprend l'étape (56) de coloriage du tissu montré dans ladite image sélectionnée (10 ; 20) avec une première couleur ou teinte et dans lequel l'étape (c) comprend l'étape (58) de coloriage du tissu montré dans les autres images (10', 10", 10'''; 22, 24, 26) dans la série, avec une couleur ou teinte contrastée.

4. Procédé selon l'une quelconque des revendications précédentes, dans lequel l'image sélectionnée (10 ; 20) est séquentiellement affichée en alignement, en chevauchement avec chacune desdites autres images (10', 10", 10'''; 22, 24, 26).

5. Procédé selon l'une quelconque des revendications précédentes, dans lequel ladite image sélectionnée (10 ; 20) est affichée statiquement tout en affichant, séquentiellement et simultanément, lesdites autres images (10', 10", 10'''; 22, 24, 26).

6. Procédé selon l'une quelconque des revendications précédentes, dans lequel le tissu se déplaçant comprend la paroi d'un coeur battant et dans lequel ladite image sélectionnée (10 ; 20) représente une vue du coeur battant à un point prédéterminé dans le cycle cardiaque.

7. Procédé selon la revendication 6, dans lequel ledit point prédéterminé dans le cycle cardiaque comprend une fin de diastole.

8. Procédé selon l'une quelconque des revendications précédentes, dans lequel lesdites séries d'images (10', 10", 10'''; 22, 24, 26) de l'étape (a) sont acquises par ultrasons.

9. Procédé selon l'une quelconque des revendications 6 à 8 comprenant de plus l'alignement sensiblement concentrique de chacune des images du coeur (10', 10" 10'''; 22, 24, 26) par rapport à au moins une autre image du coeur dans la série.

10. Procédé selon la revendication 9, dans lequel l'étape d'alignement comprend l'étape consistant à translater chacune des autres images du coeur (22, 24, 26) dans ladite série en alignement en chevauchement avec une image sélectionnée (20) parmi lesdites images du coeur (20, 22, 24, 26).

11. Procédé selon la revendication 10, dans lequel la translation de l'une (par exemple 22) desdites autres images du coeur (22, 24, 26) en alignement en chevauchement avec ladite image sélectionnée (20) parmi lesdites images du coeur (20, 22, 24, 26), influence également un alignement en chevauchement d'au moins une autre image du coeur (par exemple 24) avec ladite image sélectionnée (20) parmi lesdites images du coeur (20, 22, 24, 26).

12. Procédé selon l'une quelconque des revendications précédentes, dans lequel l'étape (a) comprend l'étape consistant à acquérir par ultrasons une série d'images à échelle de gris (10', 10", 10'''; 22, 24, 26) dudit tissu pendant une période de son activité de mouvement et comprend une autre étape consistant à sélectionner une plage de degrés d'échelle de gris qui doivent être coloriés.

13. Dispositif de traitement pour présenter des images cardiaques pour diagnostic, le dispositif comprenant :

un moyen pour acquérir, en une séquence temporelle correcte, une série d'images cardiaques (10', 10", 10'''; 22, 24, 26) pendant une période de son activité de mouvement; caractérisé en ce que le dispositif comprend de plus :
un moyen pour créer, à partir d'une image sélectionnée dans la série, une image de masque (10 ; 20) dans laquelle la paroi du coeur est représentée dans une première couleur;
un moyen pour représenter la paroi du coeur dans une pluralité d'autres images (10', 10", 10'''; 22, 24, 26) dans la série dans une seconde couleur; et
un moyen (figure 3a à 3c) pour afficher ladite image de masque tout en affichant séquentiellement, simultanément, lesdites autres images (10', 10", 10''' ; 22, 24, 26) en au moins un alignement en chevauchement partiel avec ladite image de masque.

14. Dispositif de traitement selon la revendication 13, comprenant de plus un moyen (40, 42, 44) pour déterminer les parties desdites images acquises (10', 10", 10'''; 22, 24, 26) de ladite série qui doit être représentée en couleur.

15. Dispositif de traitement selon la revendication 14, dans lequel ledit moyen pour acquérir est conçu pour acquérir une série d'images cardiaques à échelle de gris et dans lequel ledit moyen de détermination (40, 42, 44) est conçu pour désigner une plage d'intensités d'échelle de gris qui doivent être représentées en couleur.

16. Dispositif de traitement selon l'une quelconque des revendications 13 à 15, comprenant de plus un moyen pour translater la représentation de la paroi du coeur des images (22, 24, 26) ultérieures dans la série à ladite image de masque (20) en alignement avec la représentation de la paroi du coeur de ladite image de masque (20).

FIG-1a    FIG-1b    FIG-1c    FIG-1d

10    10'    10"    10'''

FIG-2a    FIG-2b    FIG-2c

10    10    10

10'    10"    10'''

EP 0 585 070 B1

FIG-3a

FIG-3b

FIG-3c

FIG-4

FIG-5a

FIG-5b

*FIG-6*

```
              ACQUIRE AN IMAGE SEQUENCE
                         │
              50         ▼
              STORE IN CINELOOP          52
                         │
                         ▼
   54       ACTIVATE COLOR PROCESSING
                         │
                         ▼
            CREATE MASK IMAGE
   56       SUPERIMPOSING OR AVERAGING
            FRAMES 1 AND 2
                         │
                         ▼
   58       PROCESS SUBSEQUENT FRAMES
            IN CONTRASTING COLOR
                         │
                         ▼
   60          OBSERVE IMAGE
                         │
                         ▼
   62                                 YES    SET
            ADJUSTMENT  ──────────────────►  GRAYSCALE
            NEEDED                           THRESHOLD
            ?
                         │ NO                   64
                         ▼
     YES                                 
            TRANSLATION
            NEEDED
            ?                    66
   TRANSLATE FRAME n  ───68      │ NO
                         
   TRANSLATE ALL
   SUBSEQUENT FRAMES  ──70
                         
              72
   NO      COMPLETED
           ALL FRAMES
           ?
              YES
                         ▼
            REVIEW PROCESSED        74
            CINELOOP
```

13